# EUROPEAN PATENT SPECIFICATION

(11) **EP 3 426 792 B1**
(45) Date of publication and mention of the grant of the patent: **29.04.2020**
(21) Application number: 17713588.6
(22) Date of filing: 03.03.2017
(51) Int. Cl.: C12P 19/30, C12N 15/10

(54) **PROCESS FOR RNA PRODUCTION**
VERFAHREN ZUR RNA-HERSTELLUNG
PROCÉDÉ DE PRODUCTION D'ARN

(30) Priority: 10.03.2016 IT UA20161550
(43) Date of publication of application: 16.01.2019
(73) Proprietor: PROSOL S.p.A., 24040 Madone (BG) (IT)
(72) Inventor: BONVICINI, Daniele, 20052 Monza (MB) (IT); LISSONI, Stefano, 24030 Mapello (BG) (IT); TOGNI, Ermanno, 24030 Presezzo (BG) (IT)
(74) Representative: Ferreccio, Rinaldo
(86) International application number: PCT/EP2017/054989
(87) International publication number: WO 2017/153266

(56) References cited:
- WO-A2-2004/067758
- JP-A- S51 106 791
- US-A- 4 623 723
- US-A- 4 649 111

## Description

### Technical Field

The present invention relates to the field of food industry and zootechnics as well as of pharmaceutical industry, in particular in the areas where the consumption or the use of good quality ribonucleic acid (RNA) is provided.

More particularly, the present invention relates to a process for the production of good quality RNA on an industrial scale, aimed to obtain products of food, pharmaceutical, and zootechnical interest.

### Background art

It is known that ribonucleic acid (RNA) is formed by a sequence of nucleotides, in which the sugar is ribose and the nitrogenous bases are adenine, guanine, cytosine and uracil. There are numerous types of RNA which show different levels of structural complexity, which correspond to different functions and roles within the cell; RNA can form extremely complex secondary and tertiary structures and recognize in a specific manner complementary sequences present both in the DNA and in other RNAs.

It is known that the ribonucleic acid does not contain the genetic information, but plays a crucial role in its transfer to the outside of the core, in protein synthesis and in regulating gene expression. Because of its many functions, the RNA is localized both at the nuclear and at the cytoplasm level.

Recently, the ribonucleic acid has become a product of interest in the pharmaceutical field, where it has been shown to have different potentials. In fact, for example, its ability to reduce wrinkle thickness, giving elasticity to the skin has been demonstrated; further, it shows an energizing effect, antioxidant, antiviral activities and it seems to have an important role in the cognitive enhancement in patients with neurodegenerative diseases.

It is also known its use as a supplement in the food industry, where it has a leading role in contributing to the rapid proliferation of cells. This feature is particularly useful following surgery, injuries and accidents.

A further use of the ribonucleic acid provides its depolymerisation and degradation to individual ribonucleotides following enzymatic or chemical treatment. The ribonucleotides, optionally in combination with specific amino acids such as glutamate, are in fact known for their properties of improving the flavour and, for this reason, they are normally added to different types of foods, including sauces and aromatic seasonings.

The use of ribonucleotides is known even in the zootechnical field, where they favour both the correct development of the younger animals and a rapid immune response against pathogens.

It is also known that the primary source to obtain important amounts of RNA is represented by microorganisms. Any type of microorganism can be used as a source of RNA, however, bacterial and fungal microorganisms are preferred, especially those already recognized by the governmental institution FDA as GRAS (generally recognized as safe) and which therefore can be used with safety for human consumption. In particular, microorganisms with a high RNA content are preferred.

Examples of microorganisms suitable to be used for this purpose comprise filamentous fungi such as *Trichoderma* and *Aspergillus* and yeasts. The most commonly used yeast strains belong to the genera *Saccharomyces*, *Kluyveromyces* or *Candida.* Among the suitable bacterial microorganisms is known the use of lactic acid bacteria, for example *Lactobacillus.*

It was proposed (patent JP 51-106791) a process for the production of RNA from yeast, comprising a step of RNA extraction from yeast and a purification and concentration step of the yeast extract by ultrafiltration.

In particular, the above-mentioned process teaches to submit the yeast extract to ultrafiltration, using membranes with a molecular weight cut-off equal to 1000-20,000 Dalton, preferably 5,000-20,000 Dalton, in order to obtain a concentrated liquid enriched in RNA. Subsequently, said liquid is acidified with hydrochloric acid to pH 2 to assist the precipitation of the ribonucleic acid.

Following the precipitation, this method provides a necessary further step of purification, since the precipitated RNA is not pure enough to be suitable for the applications in the food field illustrated in the above-mentioned patent.

The RNA containing precipitate should then be re-suspended in an alkaline solution (pH 6-8). Such a condition, in fact, allows to solubilize the RNA and promotes the precipitation of high molecular weight molecules, including the proteins that were not removed in any of the previous steps and the presence of which lowers the purity degree of the final product.

This mandatory final purification step implies additional times and costs to those of the preceding steps of said process and anyway JP 51-106791 only describes embodiments on a laboratory scale and the process described in said patent application actually appears to be poorly suited to be performed on an industrial scale.

The most common standard process which is typically applied to obtain RNA from microbial cells is characterized by multiple steps. The used microorganisms, preferably yeasts, can be in the form of inactive powder or cream or in an active form in a culture medium, which may be concentrated prior to the extraction procedure in order to reduce the volume to be treated.

In order to cause the release of the cell content, various techniques known to the expert of the art can be applied, including heating to high temperatures or total enzymatic digestion, in order to cause the rupture and disintegration of cell walls and membranes.

The soluble content that includes RNA is first separated from the insoluble cellular components by centrifugation, and the so obtained liquid is concentrated, for example 2:1, by an osmosis process, generating a retentate enriched in RNA.

Said retentate is acidified by the addition of hydrochloric acid to pH 2. In this condition, in fact, the RNA flocculates and precipitates, thus allowing its recovery by centrifugation and filtration.

The last step of this standard process consists of dissolving the RNA precipitate in a pH 6 solution, in order to release the impurities and the high molecular weight components, including proteins, which were incorporated in the RNA flocs. The second RNA precipitation in an acidic environment allows, finally, to recover the final product of interest.

The problem underlying the present invention was to provide a process particularly suitable for the application on an industrial scale and ensuring a good quality RNA production even without resorting to the final purification step provided by known procedures.

### Summary of the invention

The problem has been solved by providing a process for the production of RNA from microbial cells, which comprises steps of:
a) treating said cells to release the cell content thereof, including RNA;
b) separating the RNA present in said cell content from other soluble compounds via an osmotic process by the use of semi-permeable membranes and concentration, obtaining a retentate enriched in RNA;
c) adjusting the retentate pH to a value between 2 and 3, thus causing the precipitation of RNA, and
d) recovering the precipitated RNA by filtration or centrifugation,
characterized in that said osmotic process with the use of semi-permeable membranes is a diafiltration and that, prior to said step c) of adjusting the retentate pH to a value between 2 and 3, a salting out step b') is performed at a pH between 3.5 and 5.0 with ensuing precipitation of the proteins present in the retentate.

In one embodiment, the process also comprises a further step e) of dissolving the RNA precipitate obtained in step d) at a pH between 4.5 and 7.5, and removing any undissolved impurities at this pH, and subsequent recovery of the RNA by repeating steps c) and d).

In one aspect of the present invention the above mentioned microbial cells can be fungus, bacterium or yeast cells.

Preferably, said fungus is of the filamentous type, conveniently selected from *Trichoderma* and *Aspergillus.*

Preferably said yeast belongs to a genus selected from the group comprising *Saccharomyces*, *Kluyveromyces* or *Candida.* Even more preferably, the yeast is *Kluyveromyces.*

Preferably, said bacterium is selected from the group comprising the lactic acid bacteria. Even more preferably, the lactic bacterium is *Lactobacillus.*

In another aspect of the present invention, the above mentioned semi-permeable membranes used in the diafiltration process have a molecular weight cut-off less than or equal to 400 Dalton, preferably between 150 Dalton and 300 Dalton.

Preferably, said semi-permeable membranes are made of polysulfone.

The operating pressure to carry out the diafiltration by said membranes is preferably maintained between 8 bar and 12 bar, advantageously between 9.5 bar and 10.5 bar.

In a further aspect of the present invention, said step b' of salting out is performed in the presence of a chloride, preferably selected from the group comprising ammonium chloride, potassium chloride, sodium chloride, calcium chloride.

Preferably, the chloride is ammonium chloride.

Preferably, said chloride concentration is between 20 g/L and 120 g/L, preferably between 40 g/L and 90 g/L, even more preferably between 40 g/L and 80 g/L.

Preferably, said step b' is performed at a temperature comprised between 0 °C and 12 °C, more preferably between 3 °C and 7 °C, even more preferably between 4 °C and 6 °C.

Preferably, the time of said step b' is generally a time of at least 15 minutes, preferably between 1 and 3 hours.

In one aspect of the present invention, the above mentioned additional step e) of dissolving the RNA containing precipitate obtained in step d) at a pH between 4.5 and 7.5 is performed by adding a basic compound to an aqueous dispersion of said precipitate.

Preferably, the basic compound is a strong base, preferably an alkali or alkaline-earth hydroxide, and is advantageously selected from calcium hydroxide, sodium hydroxide and potassium hydroxide. Conveniently, the basic compound is sodium hydroxide.

The strong base is used in the form of an aqueous solution at a concentration comprised between 20% and 40%, preferably between 25% and 35%, even more preferably at a concentration of 30%.

The above percentages, like all the percentages given in the present disclosure, unless otherwise specified, are intended as weight/volume percentages.

The reaction time of said step e) is generally at least 15 minutes, preferably at least 30 minutes, conveniently from 30 to 120 minutes.

The reaction temperature of said step e) is generally maintained below 12 °C, preferably below 10 °C, even more preferably below 8 °C.

In one aspect of the present invention, the treatment of the microbial cells of step a) may be a mechanical, enzymatic or heat treatment, preferably is a heat treatment.

In this latter case, the treatment temperature is preferably maintained in a range of 65 °C-100 °C, even more preferably between 85 °C and 95 °C, conveniently it is about 90 °C, for a time comprised between 30 and 120 minutes, preferably between 40 and 70 minutes.

The RNA production process according to the present invention, differently from the processes known from the prior art, provides for a diafiltration step prior to the concentration step, in order to optimize the effectiveness of the semi-permeable membranes and expel with the permeate the salts present in the solution.

In addition, the semi-permeable membrane used for the diafiltration has the characteristic of having a porosity designed specifically for retaining the organic matrices and allow to permeate the inorganic matrices, corresponding to a cut-off of 150-400 Dalton.

The process of the present invention then advantageously has a step of salting out of the proteins, which guarantees removal thereof prior to the flocculation and the RNA precipitation step.

The effectiveness of this step is also favoured by temperature and pH control of the precipitation reaction. In fact, in the process according to the present invention, the temperature is advantageously maintained between 4 °C and 6 °C; this selected range of temperature acts as a "catalyst" of the reaction, favouring the flocculation of the proteins. Furthermore, the pH of the salting-out reaction is conveniently acid and it is maintained in a range between 4 and 5; in fact, if the pH value is less than this range, there is the risk that the RNA flocculates together to the proteins.

The excellent performance of the membranes used to carry out the diafiltration together with the step of salting-out of the proteins make the step of final purification of the precipitated RNAunnecessary, which step is instead required in the processes known from the prior art. In fact, the RNA obtained in step d) of the process of the present invention is already sufficiently pure to be used for products intended for human consumption or use in animal feed.

Further, the omission of the afore-mentioned step of final purification significantly reduces the process times, thus allowing to increase the productivity of the product of interest on an industrial scale.

### Detailed description of a preferred embodiment

Further features and advantages of the present invention will become apparent from the following description of an embodiment, given for purposes of illustration and not of limitation.

In a production reactor of an industrial plant, osmotized (i.e. reverse osmosis-treated) water and, subsequently, inactivated yeast powder, are loaded, obtaining a creamy dispersion.

The reactor is externally insulated and equipped with flowing steam heating system. The above dispersion temperature is brought to a temperature between 65 °C and 100 °C, preferably 80 °C and 90 °C and maintained for 1-2 hours. In such conditions the inactivation of possible natural enzymes of the yeast, as well as the lysis of the cell membrane and a part of the cell wall with the consequent release of the cytoplasmic and nuclear content, including RNA, are obtained.

The temperature inside the reactor is then raised to 75-80 °C by addition of cold osmotized water.

By introducing the dispersion into two centrifugal separators, the exhausted cell walls are removed from the solution giving rise to a waste referred to as "exhausted yeast" or "slurry", having a dry matter normally between 18% and 22%. In this step, the yeast is washed with osmotized water in a 1:1 weight ratio. Also, an increase of the recovered liquid volume, containing the RNA, due to the addition of osmotized water at the inlet in the centrifugal separators to improve functioning occurs.

The recovered liquid containing the RNA, with a dry weight between 3% and 4% and a pH normally between 5.3 and 5.5, deprived of cell walls, is sent to supply tanks of an osmosis plant.

This plant is equipped with polysulfone semi-permeable membranes with a nominal pore size of 150-300 Dalton, for example the membranes mod. DK8038C (trade name DK8038C Durasan* Elements, manufactured by GE Water & Process Technologies, USA), and it is programmed to perform a diafiltration treatment and a final concentration step on the incoming liquid.

During diafiltration, the RNA containing solution (stored in the supply tanks), passes through the osmosis plant and it is recirculated in the tank from which it was removed. In the supply tank a volume of osmotized water equal to the volume of the discarded permeate is continuously added. Upon completion of this operation a volume of permeate equal to that of the loaded liquid will be obtained. With the discarded permeate a portion of chloride ions, phosphate ions, potassium ions, sodium ions and sulfate ions is eliminated.

The RNA-containing diafiltered solution is then concentrated, always by the use of the osmosis plant. A RNA containing liquid, that is the retentate, and a discarded permeate are thus obtained.

The retentate, which has a temperature of about 45-50 °C and a pH between 4.3 and 4.5, is then subjected to a cooling process, by passage through four heat exchangers with decreasing temperatures and stored in a dedicated tank at temperature of 4-6.5 °C.

At this point the treatment with NH₄Cl is applied: an amount of salt variable between 20 g and 120 g, preferably between 40 g and 80 g per liter of retentate, depending on the degree of final pureness to be obtained, is added. First, this addition causes a variation of the temperature and pH conditions. The temperature undergoes a lowering of about 3 °C, due to the endothermic reaction of the salt dissolution. The pH tends to decrease, in the order of 0.5 units, reaching values comprised between 3.2 and 3.5.

After stirring for a few minutes, the liquid is sent to the precipitation tanks and left resting for a time of at least 1 hour at said temperature. During the resting step the salt causes a salting out process which affects a portion of the proteins present therein, i.e. there is a flocculation thereof, which become insoluble.

The proteins are removed by the passage of the solution through a clarifying centrifuge at continuous shooting and moving with water. For effect of this step there are obtained a precipitate of proteins, intended to be discarded, and a RNA containing liquid, which is subjected to further cooling, which brings it at temperatures between 4 °C and 6 °C, and sent back to the precipitation tanks. The final volume of the RNA containing liquid obtained from the centrifuge undergoes an increase of about 10% due to the water used for moving the product within the same, before it carries out the "shot", *i.e*. the expulsion of the material accumulated inside, which is to be discarded.

By the addition of hydrochloric acid, the pH is lowered down to a value of 2. This allows the RNA flocculation and the separation thereof from the aqueous solution. Again with the use of clarifiers, the flocculated RNA is separated, thus obtaining a 7-10% dry matter dispersion containing RNA and a liquid to be discarded. The discarded liquid in this case contains another portion of proteins that, with the addition of the salt, have been solubilized.

The quality of the thus obtained RNA is measured by UV title and normally it reaches a value of 78-80% calculated on the dry matter.

If deemed necessary for specific applications, such as certain specific applications in the pharmaceutical field, a second optional step of RNA purification, to ensure the highest quality standards of the final product, is carried out.

In this case the RNA containing dispersion is diluted with osmotized water and, by means of pH adjustment to a value between 4.5 and 7.5, preferably between 5.5 and 6.5 with NaOH, the RNA is solubilized. This procedure makes it possible to release any further residual impurities that may have remained incorporated in the floes of RNA formed with the first precipitation.

The thus obtained solution is maintained under stirring and at a controlled temperature below 8 °C for at least 15 min. Then, the precipitation process is repeated, by lowering the pH, with the addition of HCl. The passage on clarifiers is repeated, thus obtaining a new separation of the flocculated RNA from the solubilized compounds.

Following the passage on the centrifugal clarifiers, an increase of the volume of the discarded fraction occurs, due to the addition of osmotized water at the inlet of the solution in the clarifiers, in order to optimize the operation.

A RNA containing dispersion with a dry matter equal to an average of 8-10% and a liquid to be discarded that has instead a dry matter equal to 1.6-1.8%, the 4-5% thereof consisting of proteins, are obtained.

The quality of the RNA obtained with this second purification step, expressed as UV title, reaches on average value over 86% (calculated on dry matter).

### Qualitative and quantitative RNA assessments

The qualitative and quantitative aspects of the obtained product were analyzed. The RNA quality is usually measured according to two parameters: Tannhauser titer and UV titer.

The Tannhauser titer (Schmidt, G., Tannhauser, SJ, 1945. A method for the determination of DNA, RNA and phosphoproteins in animal tissues. J. Biol. Chem. 161, 83-89) indicates the amount of structurally intact RNA present in the product, it does not take into account any fragments of chain, oligonucleotides or single nucleotides that may have been generated during the process. It is used by the Applicant as a quality indicator and to estimate the yields of certain secondary processes applied on RNA. It is expressed in % on dry matter.

The UV titer indicates the amount of total RNA, including therefore any short chain RNA or oligonucleotides. It is the data that is used as reference for the marketing of the product, and that is uniquely recognized as indicative of the RNA present. It is also expressed in % of dry matter.

The amount of the RNA obtained (expressed as percentage yield) is measured as the percentage ratio between weight of the RNA (expressed in kilograms of dry matter) and the weight of the yeast used to produce it. It is easy to understand that even small percentage changes in yield in the order of tenths of a point, are significant.

The product obtained according to the present invention was compared with that obtained according to the above mentioned standard process, under the qualitative and quantitative profile, as shown in Table 1.

**Table 1: Comparison of the mean values of yield and purity expressed by UV titer and Tannhauser titer of the RNA obtained by the standard procedure versus to that obtained by the process according to the present invention. Yield > 5% indicates that the yield is normally higher than 5%, reaching a peak of 6.5%.**

| Average values according to the standard process | | | Average values according to theprocess of the present invention | | |
|---|---|---|---|---|---|
| Yield % | Tannhauser Title | UV Titer | Yield % | Tannhauser Titer | UV Titer |
| 4.70% | 61.44% | 66.57% | > 5% | 75 % | 80 % |

The results show that the RNA obtained according to the process of the present invention has both an UV titer and a Tannhauser titer higher than those obtained according to the standard process, indicating a higher purity degree of the final product.

Furthermore, also the percentage yield of RNA obtained by the process of the present invention is higher than that obtained using the standard process.

### EXAMPLE

### RNA extraction from yeast

5000 L of osmotized water, to which 1700 kg of inactivated yeast powder of the genus *Kluyveromyces* are added, are loaded into a 10000 L production reactor, obtaining a creamy dispersion.

The dispersion is brought to a temperature of 90 °C for 1 hour and then 1200 L of cold osmotized water are added into the reactor, to lower the temperature to 80 °C.

By passage through two Westphalia centrifugal separators, the first of which equipped with 2 mm nozzles and the second with 2.5 mm nozzles, a volume of exhausted cell walls equal to about 6500 L, having a dry matter of 22%, is removed. In this step, the yeast is washed with osmotized water in a 1:1 weight ratio.

### Diafiltration and concentration

The RNA containing recovered liquid, with a volume of approximately 13000 L, with a dry weight of about 3.5% and a pH of 5.4, is then sent to the supply tanks of the osmosis plant.

This plant is equipped with 27 polysulfone semi-permeable membranes DK8038C with a nominal pore size of 150-300 Dalton. At the end of this operation a volume of permeate equal to 13000 L is obtained.

The RNA containing diafiltered solution is then concentrated to a 2:1 ratio, thus obtaining 6500 L of the RNA containing retentate and 6500 L of a permeate to be discarded. The retentate, which has a temperature of about 47 °C and a pH of 4.4, is sent to the production department, where it is cooled to a temperature of 4 °C.

### Salting out of proteins and removal thereof

An amount of ammonium chloride of 42.4 g per liter of liquid is then added. The temperature undergoes a lowering of about 3 °C and the pH tends to decrease in the order of 0.5 units, reaching a value of 3.2. After stirring for 10 minutes, the liquid is sent to the precipitation tanks and left to stand for a time of at least 60 minutes, to the said temperature.

The precipitate is removed, producing a waste equal to 340 L, with a dry matter equal to 4.71%, the 46.50% of which is made up of proteins. The RNA containing liquid is cooled to 4 °C and sent back to the precipitation tanks. The final volume of RNA containing liquid obtained from the centrifuge undergoes an increase of about 10% to about 7500 L.

### RNA precipitation

Hydrochloric acid is added until a pH of 2 is obtained and, by means of clarifiers, the flocculated RNA is separated. 1000 L of RNA containing dispersion with a dry matter content of 10% and 8000 L of discarded liquid that has a dry matter content of 6.87%, the 9.56% of which consists of proteins, are thus obtained.

The quality of the thus obtained RNA is measured by UV titer and is equal to 79%.

### Optional second RNA precipitation

The dispersion, which contains RNA and has a volume of about 1000 L, is diluted in 1:1 ratio with osmotized water and, by correction of the pH to 6 with 30% NaOH, the precipitated RNA is solubilized.

The thus obtained solution is kept under stirring at less than 8 °C for at least 30 min. The precipitation process is then repeated, by lowering the pH to 2, with the addition of 30% hydrochloric acid.

The passage through the clarifiers is repeated, thus obtaining a new separation of flocculated RNA from the compounds in solution. About 700 L of RNA containing dispersion with an average dry matter of 10% and 2400 L of discarded liquid which instead has a dry matter of 1.6%, the 5% of which consists of proteins, are obtained.

The quality of the thus obtained RNA is measurable by UV titer and normally reaches a value of at least 86%.

## Claims

1. A process for the production of RNA from microbial cells, which comprises steps of:
a) treating said cells to release the cell content thereof, including RNA;
b) separating the RNA present in said cell content from other soluble compounds via an osmotic process by the use of semi-permeable membranes and concentration, obtaining a retentate enriched in RNA;
c) adjusting the retentate pH to a value between 2 and 3, thus causing the precipitation of RNA, and
d) recovering the precipitated RNA by filtration or centrifugation,
**characterized in that** said osmotic process with use of semi-permeable membranes is a diafiltration, said semi-permeable membranes having a molecular weight cut-off less than or equal to 400 Dalton, preferably between 150 and 300 Dalton, and that, prior to said step c) of adjusting the retentate pH to a value between 2 and 3, a salting out step b') is performed at a pH between 3.5 and 5.0 with ensuing precipitation of the proteins present in the retentate.

2. The process according to claim 1, wherein said process also comprises a further step e) of dissolving the precipitated RNA obtained in step d) at a pH between 4.5 and 7.5 and removing the impurities that are undissolved at such pH, and subsequently recovering RNA by repeating steps c) and d).

3. The process according to any one of claims 1 and 2, wherein said microbial cells are fungus, bacterium or yeast cells.

4. The process according to any one of claims 1-3, wherein said fungus is a filamentous fungus, conveniently selected from *Trichoderma* and *Aspergillus.*

5. The process according to any one of claims 1-3, wherein said yeast belongs to a genus selected from the group comprising *Saccharomyces, Kluyveromyces* or *Candida,* and it is preferably *Kluyveromyces.*

6. The process according to any one of claims 1-3, wherein said bacterium is selected from the group comprising lactic acid bacteria and is preferably a *Lactobacillus.*

7. The process according to claim 1, wherein said semi-permeable membranes are made of polysulfone.

8. The process according to claims 1 and 7 wherein the operating pressure to carry out the diafiltration by said membranes is preferably maintained between 8 bar and 12 bar, advantageously between 9.5 bar and 10.5 bar.

9. The process according to any one of claims 1-8, wherein said step b' of salting out is performed in the presence of a chloride, preferably selected from the group comprising ammonium chloride, potassium chloride, sodium chloride, calcium chloride, with ammonium chloride being particularly preferred.

10. The process according to claim 9, wherein said chloride concentration is between 20 g/L and 120 g/L, preferably between 40 g/L and 90 g/L, even more preferably between 40 g/L and 80 g/L.

11. The process according to claims 9 and 10, wherein said step b' is performed at a temperature comprised between 0°C and 12°C, preferably between 3°C and 7°C, more preferably between 4°C and 6°C, for at least 15 minutes, preferably between 1 and 3 hours.

12. The process according to any of claims 2-11, wherein said further step e) of dissolving the precipitated RNA obtained in step d) at a pH between 4.5 and 7.5 is performed by adding a basic compound to an aqueous dispersion of said precipitate.

13. The process according to claim 12, wherein said basic compound is a strong base, preferably an alkali or alkaline-earth hydroxide, and it is advantageously selected from sodium hydroxide, calcium hydroxide and potassium hydroxide, conveniently it is sodium hydroxide.

14. The process according to claim 13, wherein said strong base is used as an aqueous solution at a concentration comprised between 20% and 40%, preferably between 25% and 35%, even more preferably at a concentration of 30%.

15. The process according to claims 12-14, wherein the reaction time of said step e) is generally of at least 15 minutes, preferably at least 30 minutes, conveniently from 30 to 120 minutes, at a reaction temperature generally maintained below 12°C, preferably below 10°C, even more preferably below 8°C.

16. The process according to any one of claims 1-15, wherein the microbial cells treatment of step a) is a mechanical, enzymatic or heat treatment, preferably is a heat treatment.

17. The process according to claim 16, wherein said treatment is a heat treatment, in which the temperature is maintained in a range between 65°C and 100°C, preferably between 85°C and 95°C, conveniently at about 90°C, for a time comprised between 30 and 120 minutes, preferably between 40 and 70 minutes.

## Patentansprüche

1. Verfahren zur Herstellung von RNA aus Mikrobenzellen, das folgende Schritte aufweist:
a) Behandeln der Zellen, um ihren Zelleninhalt einschließlich RNA freizusetzen;
b) Abtrennen der in dem Zelleninhalt vorhandenen RNA von anderen löslichen Verbindungen mittels eines osmotischen Prozesses durch Verwendung semi-permeabler Membranen und durch Konzentration, wobei ein Retentat erhalten wird, das an RNA angereicht ist;
c) Einstellen des pH des Retentats auf einen Wert zwischen 2 und 3, was das Ausfallen von RNA bewirkt, und
d) Rückgewinnen der ausgefällten RNA durch Filtration oder Zentrifugieren,
**dadurch gekennzeichnet, dass** der osmotische Prozess unter Verwendung semipermeabler Membranen eine Diafiltration ist, wobei die semi-permeablen Membranen eine Molekülmassen-Ausschlussgrenze von weniger als oder gleich 400 Dalton, bevorzugt zwischen 150 und 300 Dalton, haben und dass vor dem Schritt c) des Einstellens des pH des Retentats auf einen Wert zwischen 2 und 3 ein Aussalzschritt b') bei einem pH zwischen 3,5 und 5,0 mit nachfolgendem Ausfällen der in dem Retentat vorhandenen Proteine durchgeführt wird.

2. Verfahren nach Anspruch 1, wobei das Verfahren auch einen weiteren Schritt e) des Auflösens der in Schritt d) erhaltenen ausgefällten RNA bei einem pH zwischen 4,5 und 7,5 und des Entfernens der Verunreinigungen, die bei einem solchen pH ungelöst sind, und ein nachfolgendes Rückgewinnen von RNA durch Wiederholen der Schritte c) und d) aufweist.

3. Verfahren nach einem der Ansprüche 1 und 2, wobei die Mikrobenzellen Pilzzellen, Bakterienzellen oder Hefezellen sind.

4. Verfahren nach einem der Ansprüche 1 bis 3, wobei der Pilz ein filamentöser Pilz ist, der günstigerweise ausgewählt wird aus Trichoderma und Aspergillus.

5. Verfahren nach einem der Ansprüche 1 bis 3, wobei die Hefe zu einer Gattung gehört, die ausgewählt wird aus der Gruppe, die Saccharomyces, Kluyveromyces oder Candida aufweist, und die bevorzugt Kluyveromyces ist.

6. Verfahren nach einem der Ansprüche 1 bis 3, wobei das Bakterium ausgewählt wird aus der Gruppe, die Milchsäurebakterien aufweist, und bevorzugt ein Lactobacillus ist.

7. Verfahren nach Anspruch 1, wobei die semi-permeablen Membranen aus Polysulfon hergestellt sind.

8. Verfahren nach den Ansprüchen 1 und 7, wobei der Arbeitsdruck zur Ausführung der Diafiltration mittels der Membranen bevorzugt zwischen 8 bar und 12 bar, vorteilhafterweise zwischen 9,5 bar und 10,5 bar, gehalten wird.

9. Verfahren nach einem der Ansprüche 1 bis 8, wobei der Schritt b' des Aussalzens durchgeführt wird in Anwesenheit eines Chlorids, das bevorzugt ausgewählt wird aus der Gruppe, die Ammoniumchlorid, Kaliumchlorid, Natriumchlorid, Calciumchlorid aufweist, wobei Ammoniumchlorid besonders bevorzugt ist.

10. Verfahren nach Anspruch 9, wobei die Chloridkonzentration zwischen 20 g/L und 120 g/L, bevorzugt zwischen 40 g/L und 90 g/L, noch bevorzugter zwischen 40 g/L und 80 g/L liegt.

11. Verfahren nach den Ansprüchen 9 und 10, wobei der Schritt b' mindestens 15 Minuten lang, bevorzugt zwischen 1 und 3 Stunden bei einer Temperatur durchgeführt wird, die zwischen 0 °C und 12 °C, bevorzugt zwischen 3 °C und 7 °C, noch bevorzugter zwischen 4 °C und 6 °C liegt.

12. Verfahren nach einem der Ansprüche 2 bis 11, wobei der weitere Schritt e) des Auflösens der in Schritt d) erhaltenen ausgefällten RNA bei einem pH zwischen 4,5 und 7,5 durchgeführt wird durch Zugeben einer basischen Verbindung zu einer wässrigen Dispersion des Präzipitats.

13. Verfahren nach Anspruch 12, wobei die basische Verbindung eine starke Base ist, bevorzugt ein Alkali- oder Erdalkalihydroxid, und vorteilhafterweise ausgewählt wird aus Natriumhydroxid, Calciumhydroxid und Kaliumhydroxid, wobei sie günstigerweise Natriumhydroxid ist.

14. Verfahren nach Anspruch 13, wobei die starke Base als eine wässrige Lösung mit einer Konzentration, die zwischen 20 % und 40 %, bevorzugt zwischen 25 % und 35 %, sogar noch bevorzugter bei einer Konzentration von 30 % liegt, verwendet wird.

15. Verfahren nach den Ansprüchen 12 bis 14, wobei die Reaktionszeit des Schrittes e) im Allgemeinen mindestens 15 Minuten, bevorzugt mindestens 30 Minuten, günstigerweise von 30 bis 120 Minuten beträgt, wobei die Reaktionstemperatur im Allgemeinen unter 12 °C, bevorzugt unter 10 °C, noch bevorzugter unter 8 °C gehalten wird.

16. Verfahren nach einem der Ansprüche 1 bis 15, wobei die Mikrobenzellen-Behandlung von Schritt a) eine mechanische, enzymatische oder Wärmebehandlung ist, bevorzugt eine Wärmebehandlung ist.

17. Verfahren nach Anspruch 16, wobei die Behandlung eine Wärmbehandlung ist, bei der die Temperatur für eine Zeit, die zwischen 30 und 120 Minuten, bevorzugt zwischen 40 und 70 Minuten, liegt, in einem Bereich zwischen 65 °C und 100 °C, bevorzugt zwischen 85 °C und 95 °C, günstigerweise bei etwa 90 °C, gehalten wird.

## Revendications

1. Un procédé pour la production d'ARN à partir de cellules microbiennes, qui comprend les étapes consistant à :
a) traiter lesdites cellules pour libérer le contenu cellulaire de celles-ci, incluant l'ARN ;
b) séparer l'ARN présent dans ledit contenu cellulaire d'autres composés solubles par un procédé osmotique en utilisant des membranes semi-perméables et une concentration, pour obtenir un rétentat enrichi en ARN ;
c) ajuster le pH du rétentat à une valeur comprise entre 2 et 3, provoquant ainsi la précipitation de l'ARN, et
d) récupérer l'ARN précipité par filtration ou centrifugation,
**caractérisé en ce que** ledit procédé osmotique avec utilisation de membranes semi-perméables est une diafiltration, lesdites membranes semi-perméables ayant une coupure de poids moléculaire inférieure ou égale à 400 Dalton, de préférence entre 150 et 300 Dalton, et **en ce que**, avant ladite étape c) d'ajustement du pH du rétentat à une valeur comprise entre 2 et 3, une étape de relargage b') est effectuée à un pH compris entre 3.5 et 5,0, avec précipitation consécutive des protéines présentes dans le rétentat.

2. Le procédé selon la revendication 1, dans lequel ledit procédé comprend également une étape supplémentaire e) consistant à dissoudre l'ARN précipité obtenu à l'étape d) à un pH compris entre 4,5 et 7,5 et à éliminer les impuretés qui ne sont pas dissoutes à un tel pH, et à récupérer ensuite l'ARN en répétant les étapes c) et d).

3. Le procédé selon l'une quelconque des revendications 1 et 2, dans lequel lesdites cellules microbiennes sont des cellules de champignon, de bactérie ou de levure.

4. Le procédé selon l'une quelconque des revendications 1 à 3, dans lequel ledit champignon est un champignon filamenteux, commodément choisi parmi *Trichoderma* et *Aspergillus.*

5. Le procédé selon l'une quelconque des revendications 1 à 3, dans lequel ladite levure appartient à un genre choisi dans le groupe comprenant *Saccharomyces, Kluyveromyces* ou *Candida,* et c'est de préférence *Kluyveromyces.*

6. Le procédé selon l'une quelconque des revendications 1 à 3, dans lequel ladite bactérie est choisie dans le groupe comprenant les bactéries d'acide lactique et est de préférence un *Lactobacillus.*

7. Le procédé selon la revendication 1, dans lequel lesdites membranes semi-perméables sont faites de polysulfone.

8. Le procédé selon les revendications 1 et 7, dans lequel la pression de fonctionnement pour effectuer la diafiltration par lesdites membranes est de préférence maintenue entre 8 bar et 12 bar, avantageusement entre 9,5 bar et 10,5 bar.

9. Le procédé selon l'une quelconque des revendications 1 à 8, dans lequel ladite étape b' de relargage est réalisée en présence d'un chlorure, de préférence choisi dans le groupe comprenant le chlorure d'ammonium, le chlorure de potassium, le chlorure de sodium, le chlorure de calcium, le chlorure d'ammonium étant particulièrement préféré.

10. Le procédé selon la revendication 9, dans lequel ladite concentration en chlorure est comprise entre 20 g/L et 120 g/L, de préférence entre 40 g/L et 90 g/L, de façon encore préférée entre 40 g/L et 80 g/L.

11. Le procédé selon les revendications 9 et 10, dans lequel ladite étape b' est réalisée à une température comprise entre 0°C et 12°C, de préférence entre 3°C et 7°C, de façon encore préférée entre 4°C et 6°C, pendant au moins 15 minutes, de préférence entre 1 et 3 heures.

12. Le procédé selon l'une quelconque des revendications 2 à 11, dans lequel ladite étape supplémentaire e) de dissolution de l'ARN précipité obtenu dans l'étape d) à un pH compris entre 4,5 et 7,5 est réalisée en ajoutant un composé basique à une dispersion aqueuse dudit précipité.

13. Le procédé selon la revendication 12, dans lequel ledit composé basique est une base forte, de préférence un hydroxyde alcalin ou alcalino-terreux, et il est avantageusement choisi parmi l'hydroxyde de sodium, l'hydroxyde de calcium et l'hydroxyde de potassium, commodément il s'agit d'hydroxyde de sodium.

14. Le procédé selon la revendication 13, dans lequel ladite base forte est utilisée sous forme de solution aqueuse à une concentration comprise entre 20% et 40%, de préférence entre 25% et 35%, de façon encore préférée à une concentration de 30%.

15. Le procédé selon la revendication 12 à 14, dans lequel le temps de réaction de ladite étape e) est généralement d'au moins 15 minutes, de préférence d'au moins 30 minutes, commodément de 30 à 120 minutes, à une température de réaction généralement maintenue en dessous de 12°C, de préférence en dessous de 10°C, de façon encore préférée en dessous de 8°C.

16. Le procédé selon l'une quelconque des revendications 1 à 15, dans lequel le traitement des cellules microbiennes de l'étape a) est un traitement mécanique, enzymatique ou thermique, de préférence un traitement thermique.

17. Le procédé selon la revendication 16, dans lequel ledit traitement est un traitement thermique, dans lequel la température est maintenue dans une gamme allant de 65°C et 100°C, de préférence de 85°C à 95°C, de manière pratique à environ 90°C, pendant une durée comprise entre 30 et 120 minutes, de préférence allant de 40 à 70 minutes.
